# EUROPÄISCHE PATENTSCHRIFT

(11) **EP 1 188 753 B1**
(45) Veröffentlichungstag und Bekanntmachung des Hinweises auf die Patenterteilung: **24.09.2003**
(21) Anmeldenummer: 01122525.7
(22) Anmeldetag: 16.05.1997
(51) Int. Cl.: C07D 307/82

(54) **Zwischenprodukte zur Herstellung von 3-(1-Hydroxyphenyl-1-alkoximinomethyl)dioxazinen**
Intermediates in the preparation of 3-(1-hydroxyphenyl-1-alkoximinomethyl)dioxazines
Produits intermédiaires pour la préparation de 3-(1-hydroxyphényl-1-alcoximinométhyl)dioxazines

(30) Priorität: 30.05.1996 DE 19621696; 09.12.1996 DE 19651034; 19.02.1997 DE 19706399
(43) Veröffentlichungstag der Anmeldung: 20.03.2002
(62) Teilanmeldung aus: 97923924.1
(73) Patentinhaber: Bayer CropScience AG, 40789 Monheim (DE)
(72) Erfinder: Gayer, Herbert, Dr., 40789 Monheim (DE); Gallenkamp, Bernd, Dr., 42113 Wuppertal (DE); Gerdes, Peter, Dr., 52080 Aachen (DE); Heinemann, Ulrich, Dr., 42799 Leichlingen (DE); Krüger, Bernd-Wieland, Dr., 51467 Bergisch Gladbach (DE); Lantzsch, Reinhard, Dr., 42115 Wuppertal (DE); Seitz, Thomas, Dr., 40764 Langenfeld (DE); Stelzer, Uwe, Dr., 51399 Burscheid (DE)

(56) Entgegenhaltungen:
- EP-A- 0 050 283

## Beschreibung

Die vorliegende Erfindung betrifft mehrere neue Verfahren und neue Zwischenprodukte zur Herstellung von 3-(1-Hydroxyphenyl-1-alkoximinomethyl)dioxazinen, die als Vorprodukte zur Herstellung von Verbindungen mit fungiziden Eigenschaften bekannt sind (WO 95-04728).

Es ist bereits bekannt geworden, dass sich bestimmte 3-(1-Hydroxyphenyl-1-alkoximinomethyl)dioxazine ausgehend von den entsprechenden Hydroxyphenylessigsäureestem synthetisieren lassen (vgl. WO 95-04728). So erhält man z. B. (5,6-Dihydro-[1,4,2]dioxazin-3-yl)-(2-hydroxy-phenyl)-methanon-O-methyl-oxim (1), indem man Hydroxyphenylessigsäuremethylester (a) mit Dihydropyran umsetzt, den dabei entstehenden Dihydropyranylether (b) mit t-Butylnitrit in den 2-[2-(Tetrahydropyran-2-yloxy)-phenyl]-2-hydroximino-essigsäuremethylester (c) überführt, diese Verbindung mit Iodmethan zu 2-[2-(Tetrahydropyran-2-yloxy)-phenyl]-2-methoximino-essigsäuremethylester (d) alkyliert, diesen mit Hydroxylamin zu 2-[2-(Tetrahydropyran-2-yloxy)-phenyl]-2-methoximino-N-hydroxyacetamid (e) umsetzt, letzteres mit Dibromethan zu 3-{1-[2-(Tetrahydropyran-2-yloxy)-phenyl]-1-methoximino-methyl}-5,6-dihydro-1,4,2-dioxazin (f) cyclisiert, und zum Schluss die Tetrahydropyranylgruppe säurekatalysiert abspaltet. Diese Synthese kann durch das folgende Formelschema veranschaulicht werden:

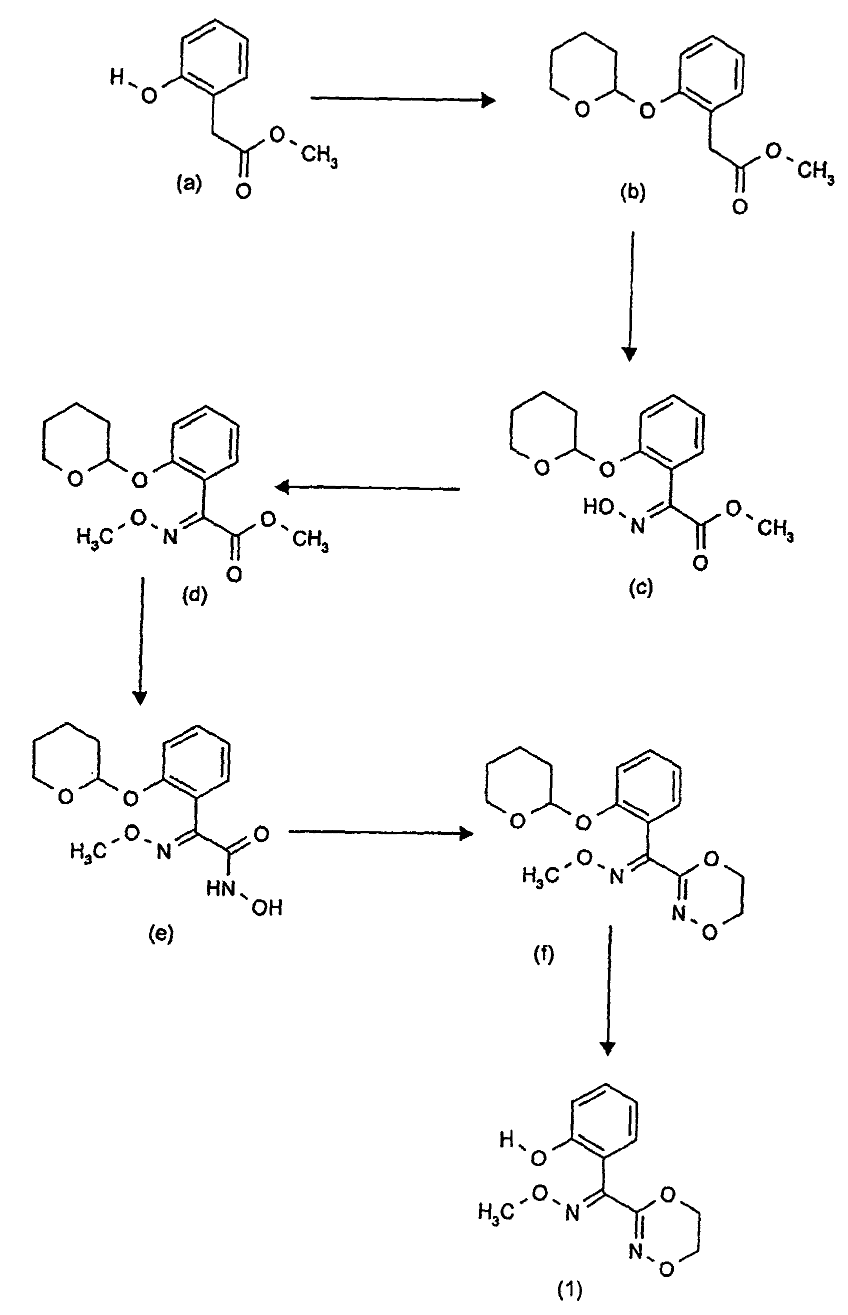

Ein entscheidender Nachteil dieses Verfahrens besteht darin, dass viele Reaktionsschritte mit teilweise geringen Ausbeuten erforderlich sind, was die Wirtschaftlichkeit dieses Verfahrens entscheidend beeinträchtigt.

Es wurde nun gefunden, dass man 3-(1-Hydroxyphenyl-1-alkoximinomethyl)dioxazine der allgemeinen Formel 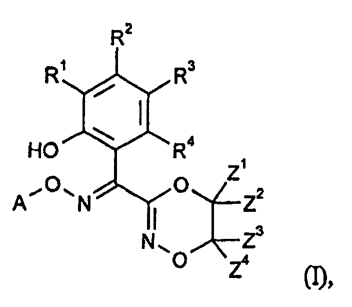 in welcher
- A: für Alkyl steht,
- R¹, R², R³ und R⁴: gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen und
- Z¹, Z², Z³ und Z⁴: gleich oder verschieden sind und unabhängig voneinander für Wasserstoff, Alkyl, Halogenalkyl oder Hydroxyalkyl stehen, oder
- Z¹ und Z² oder Z¹ und Z³ oder Z³ und Z⁴: gemeinsam mit den jeweiligen Kohlenstoffatomen, an die sie gebunden sind, einen cycloaliphatischen Ring bilden,
erhält, wenn man
a) O-Hydroxyethyl-O'-methyl-benzofurandiondioxime der Formel 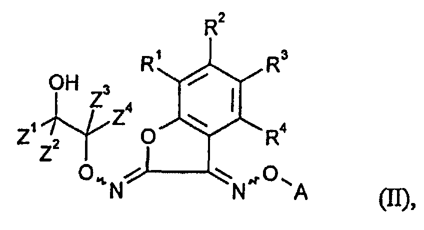 in welcher
   - A, R¹, R², R³, R⁴, Z¹, Z², Z³ und Z⁴: die oben angegebenen Bedeutungen haben,
   gegebenenfalls in Gegenwart eines Verdünnungsmittels und gegebenenfalls in Gegenwart einer Säure oder einer Base, umlagert, oder wenn man
b) Hydroxybenzoyldioxazine der Formel 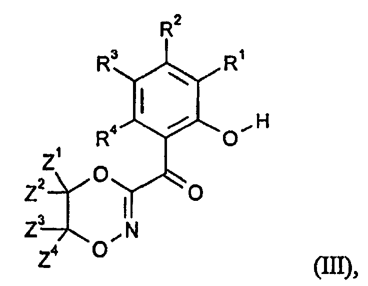 in welcher
   - R¹, R², R³, R⁴, Z¹, Z², Z³ und Z⁴: die oben angegebenen Bedeutungen haben,
   mit einem Alkoxyamin der Formel

   A-O-NH₂ (IV)

   in welcher
   - A: die oben angegebene Bedeutung hat,
   - oder einem Säureadditionskomplex davon -
   gegebenenfalls in Gegenwart eines Verdünnungsmittel und gegebenenfalls in Gegenwart eines Säureakzeptors, umsetzt, oder wenn man
c) Hydroxyphenyl-hydroximinomethyl-dioxazine der Formel 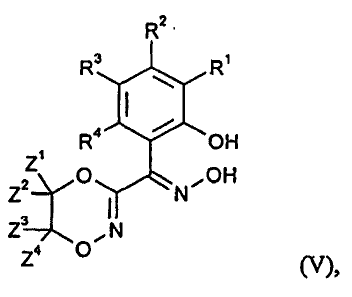 in welcher
   - R¹, R², R³, R⁴, Z¹, Z², Z³ und Z⁴: die oben angegebenen Bedeutungen haben,
   mit einem Alkylierungsmittel der Formel

   A-X (VI)

   in welcher
   - A: die oben angegebene Bedeutung hat und
   - X: für Halogen, Alkylsulfonyloxy, Alkoxysulfonyloxy oder Arylsulfonyloxy steht,
gegebenenfalls in Gegenwart eines Verdünnungsmittels, und gegebenenfalls in Gegenwart einer Base, umsetzt.

In den Definitionen sind die gesättigten oder ungesättigten Kohlenwasserstoffketten, wie Alkyl, auch in Verknüpfung mit Heteroatomen, wie in Alkoxy oder Alkylthio, jeweils geradkettig oder verzweigt.

Nach den erfindungsgemäßen Verfahren a-c) werden bevorzugt Verbindungen der Formel (I) hergestellt, in welcher
- A: für Methyl, Ethyl, n-, oder i-Propyl steht,
- R¹, R², R³ und R⁴,: gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen, steht,
- Z¹, Z², Z³ und Z⁴: gleich oder verschieden sind und unabhängig voneinander für Wasserstoff, Alkyl oder Hydroxyalkyl mit 1 bis 4 Kohlenstoffatomen oder Halogenalkyl mit 1 bis 4 Kohlenstoffatomen und 1 bis 5 gleichen oder verschiedenen Halogenatomen stehen, oder
- Z¹ und Z² oder Z¹ und Z³ oder Z³ und Z⁴: gemeinsam mit den jeweiligen Kohlenstoffatomen, an die sie gebunden sind, einen cycloaliphatischen Ring mit fünf, sechs oder sieben Kohlenstoffatomen bilden.

Besonders bevorzugt ist die Herstellung von Verbindungen der Formel (I), in welcher
- A: für Methyl oder Ethyl steht,
- R¹, R², R³ und R⁴: gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen, und
- Z¹, Z², Z³ und Z⁴: gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
- Z¹ und Z² oder Z¹ und Z³ oder Z³ und Z⁴: gemeinsam mit den jeweiligen Kohlenstoffatomen, an die sie gebunden sind, einen cycloaliphatischen Ring mit fünf, sechs oder sieben Kohlenstoffatomen bilden.

Die zur Durchführung des erfindungsgemäßen Verfahrens a) als Ausgangsstoffe benötigten O-Hydroxyethyl-O'-methyl-benzofurandiondioxime sind durch die Formel (II) allgemein definiert. In dieser Formel (II) haben A, R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³, R⁴, Z¹, Z², Z³ und Z⁴ angegeben wurden.

Die Ausgangsstoffe der Formel (II) sind noch nicht bekannt und als neue Stoffe ebenfalls Gegenstand der vorliegenden Anmeldung.

Die O-Hydroxyethyl-O'-methyl-benzofürandiondioxime der Formel (II) werden erhalten, wenn man

Verfahren d) O-Hydroxyethyl-benzofurandionmonooxime der Formel 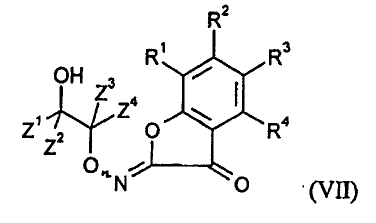 in welcher
- R¹, R², R³, R⁴, Z¹, Z², Z³ und Z⁴: die oben angegebenen Bedeutungen haben,
mit einem Alkoxyamin der Formel (IV)
- oder einem Säureadditionskomplex davon -
gegebenenfalls in Gegenwart eines Verdünnungsmittel und gegebenenfalls in Gegenwart eines Säureakzeptors, umsetzt, oder wenn man

Verfahren e) O-Alkyl-benzofurandiondioxime der Formel 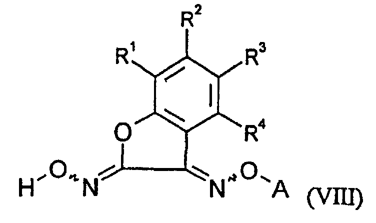 in welcher
- A, R¹, R², R³ und R⁴: die oben angegebenen Bedeutungen haben,
mit einem Ethanderivat der Formel 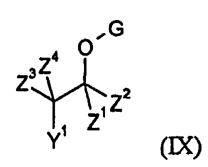 in welcher
- Y¹: für Halogen, Alkylsulfonyloxy, Arylsulfonyloxy oder Alkanoyloxy steht und
- G: für Wasserstoff steht, oder
- Y¹ und G: durch eine Einfachbindung miteinander verbunden sind, wobei
- Y¹: für Sauerstoff steht und
- G: für 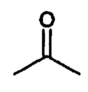 steht, oder
- Y¹ und G: gemeinsam für eine Einfachbindung stehen und
- Z¹, Z², Z³ und Z⁴: die oben angegebenen Bedeutungen haben,
gegebenenfalls in Gegenwart eines Verdünnungsmittel, und gegebenenfalls in Gegenwart einer Base, umsetzt, oder wenn man

Verfahren f) O-Hydroxyethyl-benzofurandiondioxime der Formel 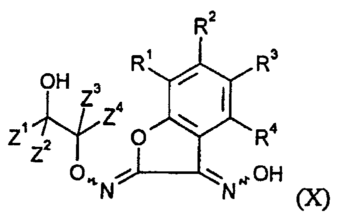 in welcher
- R¹, R², R³, R⁴, Z¹, Z², Z³ und Z⁴: die oben angegebenen Bedeutungen haben,
mit einem Alkylierungsmittel der Formel (VI),
gegebenenfalls in Gegenwart eines Verdünnungsmittels, und gegebenenfalls in Gegenwart einer Base, umsetzt oder wenn man

Verfahren m) O-Oxyethyl-O'-methyl-benzofurandiondioxime der Formel 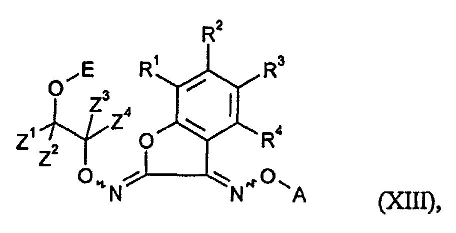 in welcher
- A, R¹, R², R³, R⁴, Z¹, Z², Z³ und Z⁴: die oben angegebenen Bedeutungen haben und
- E: für eine Acylgruppe oder eine ketalische Schutzgruppe steht,
mit Wasser oder einem Alkohol, gegebenenfalls in Gegenwart eines Veidünnungsmittels und gegebenenfalls in Gegenwart einer Säure oder einer Base, umsetzt.

Die zur Durchführung des erfindungsgemäßen Verfahrens d) als Ausgangsstoffe benötigten O-Hydroxyethyl-benzofurandionmonooxime sind durch die Formel (VII) allgemein definiert. In dieser Formel (VII) haben R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurden.

Die Ausgangsstoffe der Formel (VII) sind noch nicht bekannt und als neue Stoffe ebenfalls Gegenstand der vorliegenden Anmeldung.

Die O-Hydroxyethyl-benzofurandionmonooxime der Formel (VII) werden erhalten, wenn man

Verfahren g) Benzofurandionmonooxime der Formel 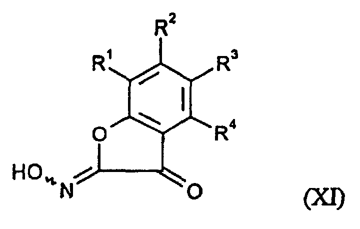 in welcher
- R¹, R², R³ und R⁴: die oben angegebenen Bedeutungen haben,
mit einem Ethanderivat der Formel (IX),
gegebenenfalls in Gegenwart eines Verdünnungsmittels, und gegebenenfalls in Gegenwart einer Base, umsetzt, oder wenn man

Verfahren n) O-Oxyethyl-benzofurandionmonooxime der Formel 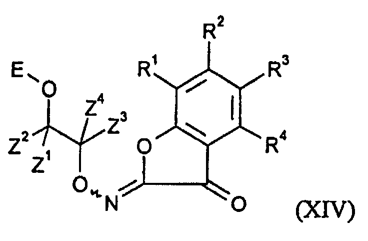 in welcher
- E, R¹, R², R³, R⁴, Z¹, Z², Z³ und Z⁴: die oben angegebenen Bedeutungen haben,
mit Wasser oder einem Alkohol, gegebenenfalls in Gegenwart eines Verdünnungsmittels und gegebenenfalls in Gegenwart einer Säure oder einer Base, umsetzt.

Die zur Durchführung des erfindungsgemäßen Verfahrens g) als Ausgangsstoffe benötigten Benzofurandionmonooxime sind durch die Formel (XI) allgemein definiert. In dieser Formel (XI) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Benzofurandionmonooxime der Formel (XI) sind bekannt und können nach bekannten Verfahren hergestellt werden (vergleiche Beilstein, E (II) 17, 462; Mameli, G. 56, 768; Chem. Ber. 35 (1902), 1640-1646; Proc. Indian Acad. Sci. Sect. A (1976) 83A(6), 238-242)).

Die zur Durchführung des erfindungsgemäßen Verfahrens n) als Ausgangsstoffe benötigten O-Oxyethyl-benzofurandionmonooxime sind durch die Formel (XIV) allgemein definiert. In dieser Formel (XIV) haben R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurden. E hat vorzugsweise bzw. insbesondere diejenige Bedeutung, die weiter unten im Zusammenhang mit der Beschreibung der erfindungsgemäßen Verbindungen der Formel (XIII) als bevorzugt bzw. als insbesondere bevorzugt für E angegeben wird.

Die Ausgangsstoffe der Formel (XIV) sind noch nicht bekannt und als neue Stoffe ebenfalls Gegenstand der vorliegenden Anmeldung.

Die O-Oxyethyl-benzofurandionmonooxime der Formel (XIV) werden erhalten, wenn man

Verfahren o) Benzofurandionmonooxime der Formel (XI) mit einem Ethanolderivat der Formel 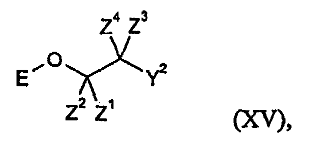 in welcher
- E, Z¹, Z², Z³ und Z⁴: die oben angegebenen Bedeutungen haben und
- Y²: für Halogen, Alkylsulfonyloxy, Arylsulfonyloxy oder Alkanoyloxy steht,
gegebenenfalls in Gegenwart eines Verdünnungsmittels und gegebenenfalls in Gegenwart eines Säureakzeptors, umsetzt.

Die zur Durchführung des erfindungsgemäßen Verfahrens o) als Ausgangsstoffe benötigten Benzofurandionmonooxime der Formel (XI) sind bereits bei der Beschreibung des erfindungsgemäßen Verfahrens g) beschrieben worden.

Die zur Durchführung des erfindungsgemäßen Verfahrens e) als Ausgangsstoffe benötigten O-Alkyl-benzofurandiondioxime sind durch die Formel (VIII) allgemein definiert. In dieser Formel (VIII) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Ausgangsstoffe der Formel (VIII) sind noch nicht bekannt und als neue Stoffe ebenfalls Gegenstand der vorliegenden Anmeldung.

Die O-Hydroxyethyl-benzofurandionmonooxime der Formel (VIII) werden erhalten, wenn man

Verfahren h) Benzofurandionmonooxime der Formel (XI) mit einem Alkoxyamin der Formel (IV),
gegebenenfalls in Gegenwart eines Verdünnungsmittels und gegebenenfalls in Gegenwart eines Säureakzeptors, umsetzt, oder wenn man
Verfahren p) Benzofurandiondioxime der Formel 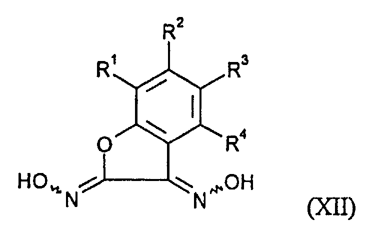 in welcher
- R¹, R², R³ und R⁴: die oben angegebenen Bedeutungen haben,
mit einem Alkylierungsmittel der Formel (VI),
gegebenenfalls in Gegenwart eines Verdünnungsmittels, und gegebenenfalls in Gegenwart einer Base, umsetzt.

Die zur Durchführung des erfindungsgemäßen Verfahrens h) als Ausgangsstoffe benötigten Benzofurandionmonooxime der Formel (XI) sind bereits bei der Beschreibung des erfindungsgemäßen Verfahrens g) beschrieben worden.

Die zur Durchführung des erfindungsgemäßen Verfahrens p) als Ausgangsstoffe benötigten Benzofurandiondioxime sind durch die Formel (XII) allgemein definiert. In dieser Formel (XII) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Benzofurandiondioxime der Formel (XII) sind bekannt und können nach bekannten Verfahren hergestellt werden (vergleiche Chem. Ber. 42 (1909), 202).

Die zur Durchführung des erfindungsgemäßen Verfahrens f) als Ausgangsstoffe benötigten O-Hydroxyethyl-benzofurandiondioxime sind durch die Formel (X) allgemein definiert. In dieser Formel (X) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Ausgangsstoffe der Formel (X) sind noch nicht bekannt und als neue Stoffe ebenfalls Gegenstand der vorliegenden Anmeldung.

Die O-Hydroxyethyl-benzofurandiondioxime der Formel (X) werden erhalten, wenn man
Verfahren i) O-Hydroxyethyl-benzofurandionmonooxime der Formel (VII),
mit Hydroxylamin - oder einem Säureadditionskomplex davon -
gegebenenfalls in Gegenwart eines Verdünnungsmittels und gegebenenfalls in Gegenwart eines Säureakzeptors, umsetzt, oder wenn man
Verfahren q) O-Oxyethyl-benzofurandiondioxime der Formel 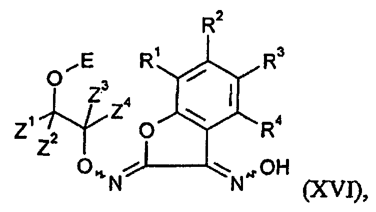 in welcher
- E, R¹, R², R³, R⁴, Z¹, Z², Z³ und Z⁴: die oben angegebenen Bedeutungen haben,
mit Wasser oder einem Alkohol, gegebenenfalls in Gegenwart eines Verdünnungsmittels und gegebenenfalls in Gegenwart einer Säure oder einer Base, umsetzt.

Die zur Durchführung des erfindungsgemäßen Verfahrens i) als Ausgangsstoffe benötigten O-Hydroxyethyl-benzofurandionmonooxime der Formel (VII) sind bereits bei der Beschreibung des erfindungsgemäßen Verfahrens d) beschrieben worden.

Die zur Durchführung des erfindungsgemäßen Verfahrens q) als Ausgangsstoffe benötigten O-Oxyethyl-benzofurandiondioxime sind durch die Formel (XVI) allgemein definiert. In dieser Formel (XVI) haben R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurde. E hat vorzugsweise bzw. insbesondere diejenigen Bedeutungen, die weiter unten im Zusammenhang mit der Beschreibung der erfindungsgemäßen Verbindungen der Formel (XIII) als bevorzugt bzw. als insbesondere bevorzugt für E angegeben werden.

Die Ausgangsstoffe der Formel (XVI) sind noch nicht bekannt und als neue Stoffe ebenfalls Gegenstand der vorliegenden Anmeldung.

Die O-Oxyethyl-benzofurandiondioxime der Formel (XVI) werden erhalten, wenn man
Verfahren r) O-Oxyethyl-benzofurandionmonooxime der Formel (XIV) mit Hydroxylamin - oder einem Säureadditionskomplex davon -
gegebenenfalls in Gegenwart eines Verdünnungsmittels und gegebenenfalls in Gegenwart eines Säureakzeptors, umsetzt.

Die zur Durchführung des erfindungsgemäßen Verfahrens r) als Ausgangsstoffe benötigten O-Oxyethyl-benzofurandionmonooxime der Formel (XIV) sind bereits bei der Beschreibung des erfindungsgemäßen Verfahrens n) beschrieben worden.

Die zur Durchführung des erfindungsgemäßen Verfahrens m) als Ausgangsstoffe benötigten O-Oxyethyl-O'-methyl-benzofurandiondioxime der Formel (XIII), sind durch die Formel (III) allgemein definiert. In dieser Formel (XIII) haben A, R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurden. E steht für eine Acylgruppe, vorzugsweise für Formyl, Acetyl oder Benzoyl, oder eine ketalische Schutzgruppe, vorzugsweise für 2-Tetrahydropyranyl, 1-Methoxy-1-ethyl, 1-Ethoxy-1-ethyl, Methoxymethyl oder Ethoxymethyl.

Die Ausgangsstoffe der Formel (XIII) sind noch nicht bekannt und als neue Stoffe ebenfalls Gegenstand der vorliegenden Anmeldung.

Die O-Oxyethyl-O'-methyl-benzofurandiondioxime der Formel (XIII) werden erhalten, wenn man
Verfahren s) O-Oxyethyl-benzofurandionmonooxime der Formel (XIV) mit einem Alkoxyamin der Formel (IV)
- oder einem Säureadditionskomplex davon -
gegebenenfalls in Gegenwart eines Verdünnungsmittel und gegebenenfalls in Gegenwart eines Säureakzeptors, umsetzt, oder wenn man

Verfahren t) O-Oxyethyl-benzofurandiondioxime der Formel (XVI) mit einem Alkylierungsmittel der Formel (VI),
gegebenenfalls in Gegenwart eines Verdünnungsmittels, und gegebenenfalls in Gegenwart einer Base, umsetzt oder wenn man
Verfahren u) O-Alkyl-benzofurandiondioxime der Formel (VIII) mit einem Ethanolderivat der Formel (XV),
gegebenenfalls in Gegenwart eines Verdünnungsmittels und gegebenenfalls in Gegenwart eines Säureakzeptors, umsetzt.

Die zur Durchführung des erfindungsgemäßen Verfahrens s) als Ausgangsstoffe benötigten O-Oxyethyl-benzofarandionmonooxime der Formel (XIV) sind bereits bei der Beschreibung des erfindungsgemäßen Verfahrens n) beschrieben worden.

Die zur Durchführung des erfindungsgemäßen Verfahrens t) als Ausgangsstoffe benötigten O-Oxyethyl-benzofurandiondioxime der Formel (XVI) sind bereits bei der Beschreibung des erfindungsgemäßen Verfahrens q) beschrieben worden.

Die zur Durchführung des erfindungsgemäßen Verfahrens u) als Ausgangsstoffe benötigten O-Alkyl-benzofurandiondioxime der Formel (VIII) sind bereits bei der Beschreibung des erfindungsgemäßen Verfahrens e) beschrieben worden.

Die zur Durchführung des erfindungsgemäßen Verfahrens b) als Ausgangsstoffe benötigten Hydroxybenzoyldioxazine sind durch die Formel (III) allgemein definiert. In dieser Formel (III) haben R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurden.

Die Ausgangsstoffe der Formel (III) sind noch nicht bekannt und als neue Stoffe ebenfalls Gegenstand der vorliegenden Anmeldung.

Die Hydroxybenzoyldioxazine der Formel (III) werden erhalten, wenn man
Verfahren k) O-Hydroxyethyl-benzofurandionmonooxime der Formel (VII),
gegebenenfalls in Gegenwart eines Verdünnungsmittels und gegebenenfalls in Gegenwart einer Säure oder einer Base, umsetzt.

Die zur Durchführung des erfindungsgemäßen Verfahrens k) als Ausgangsstoffe benötigten O-Hydroxyethyl-benzofurandionmonooxime der Formel (VII) sind bereits bei der Beschreibung des erfindungsgemäßen Verfahrens d) beschrieben worden.

Die zur Durchführung des erfindungsgemäßen Verfahrens c) als Ausgangsstoffe benötigten Hydroxyphenyl-hydroximinomethyl-dioxazine sind durch die Formel (V) allgemein definiert. In dieser Formel (V) haben R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³, R⁴, Z¹, Z², Z³ und Z⁴ angegeben wurden.

Die Ausgangsstoffe der Formel (V) sind noch nicht bekannt und als neue Stoffe ebenfalls Gegenstand der vorliegenden Anmeldung.

Die Hydroxyphenyl-hydroximinomethyl-dioxazine der Formel (V) werden erhalten, wenn man
Verfahren 1) Hydroxybenzoyldioxazine der Formel (III)
mit Hydroxylamin - oder einem Säureadditionskomplex davon -
gegebenenfalls in Gegenwart eines Verdünnungsmittels und gegebenenfalls in Gegenwart eines Säureakzeptors, umsetzt, oder wenn man

Verfahren v) O-Hydroxyethyl-benzofurandiondioxime der Formel (X)
gegebenenfalls in Gegenwart eines Verdünnungsmittels und gegebenenfalls in Gegenwart einer Säure oder einer Base, umlagert.

Die zur Durchführung des erfindungsgemäßen Verfahrens 1) als Ausgangsstoffe benötigten Hydroxybenzoyldioxazine der Formel (III) sind bereits bei der Beschreibung des erfindungsgemäßen Verfahrens b) beschrieben worden.

Die zur Durchführung des erfindungsgemäßen Verfahrens v) als Ausgangsstoffe benötigten O-Hydroxyethyl-benzofurandiondioxime der Formel (X) sind bereits bei der Beschreibung des erfindungsgemäßen Verfahrens f) beschrieben worden.

Die weiterhin zur Durchführung der erfindungsgemäßen Verfahren b), d), h) und s) als Ausgangsstoffe benötigten Alkoxyamine sind durch die Formel (IV) allgemein definiert. In dieser Formel (IV) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. Bevorzugte Säureadditionskomplexe der Alkoxyamine der Formel (IV) sind deren Hydrochloride, Sulfate und Hydrogensulfate.

Die Alkoxyamine der Formel (IV) und deren Säureadditionskomplexe sind bekannte Synthesechemikalien.

Die weiterhin zur Durchführung der erfindungsgemäßen Verfahren c), f), p) und t) als Ausgangsstoffe benötigten Alkylierungsmittel sind durch die Formel (VI) allgemein definiert. In dieser Formel (VI) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. X steht für Halogen, vorzugsweise Chlor, Brom oder Iod, Alkylsulfonyloxy, vorzugsweise Methylsulfonyloxy, Alkoxysulfonyloxy, vorzugsweise Methoxysulfonyloxy, oder Arylsulfonyloxy, vorzugsweise 4-Tolylsulfonyloxy.

Die Alkylierungsmittel der Formel (VI) sind bekannte Synthesechemikalien.

Die weiterhin zur Durchführung der erfindungsgemäßen Verfahren e) und g) als Ausgangsstoffe benötigten Ethanderivate sind durch die Formel (IX) allgemein definiert. In dieser Formel (IX) haben Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für Z¹, Z², Z³ und Z⁴ angegeben wurden. Y¹ steht für Halogen, vorzugsweise Chlor, Brom oder Iod, Alkylsulfonyloxy, vorzugsweise Methylsulfonyloxy, Arylsulfonyloxy, vorzugsweise 4-Tolylsulfonyloxy, oder Alkanoyloxy, vorzugsweise Acetyloxy. G steht für Wasserstoff oder ist durch eine Einfachbindung mit Y¹ verbunden, wobei Y¹ für Sauerstoff steht und G für Carbonyl steht, oder G steht gemeinsam mit Y¹ auch für eine Einfachbindung.

Die Ethanderivate der Formel (IX), sind bekannte Synthesechemikalien.

Das weiterhin zur Durchführung der erfindungsgemäßen Verfahren i), l) und r) als Ausgangsstoff benötigte Hydroxylamin, oder seine Säureadditionskomplexe, vorzugsweise sein Hydrochlorid, Sulfat und Hydrogensulfat, sind bekannte Synthesechemikalien.

Die weiterhin zur Durchführung der erfindungsgemäßen Verfahren o) und u) als Ausgangsstoffe benötigten Ethanolderivate sind durch die Formel (XV) allgemein definiert. In dieser Formel (XV) haben Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für Z¹, Z², Z³ und Z⁴ angegeben wurden. E hat vorzugsweise bzw. insbesondere diejenigen Bedeutung, die bereits im Zusammenhang mit der Beschreibung der erfindungsgemäßen Verbindungen der Formel (XIII) als bevorzugt bzw. als insbesondere bevorzugt für E angegeben wurde.

Y² steht für Halogen, vorzugsweise Chlor, Brom oder Iod, Alkylsulfonyloxy, vorzugsweise Methylsulfonyloxy, Arylsulfonyloxy, vorzugsweise 4-Tolylsulfonyloxy, oder Alkanoyloxy, vorzugsweise Acetyloxy.

Die Ethanolderivate der Formel (XV) sind bekannt und können nach bekannten Methoden hergestellt werden (vergleiche z. B. Newkome, George R.; Marston, Charles R., J.Org.Chem., 50, 22, 1985, 4238-4245; Henry, Chem.Ber., 7 <1874>,70)

Werden die erfindungsgemäßen Verfahren a), k) und v) in Gegenwart einer Säure durchgeführt, kommen als Verdünnungsmittel alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Dioxan, 1,2- Dimethoxyethan, 1,2-Diethoxyethan oder Anisol; Ester wie Essigsäuremethylester oder Essigsäureethylester, oder Sulfone, wie Sulfolan sowie beliebige Mischungen der genannten Verdünnungsmittel. Besonders bevorzugte Verdünnungsmittel sind Ether, wie Diethylether, 1,2-Diethoxyethan oder Anisol; aromatische Kohlenwasserstoffe, wie beispielsweise Benzol, Toluol oder Xylol.

Werden die erfindungsgemäßen Verfahren a), k) und v) in Gegenwart einer Base durchgeführt, kommen als Verdünnungsmittel Wasser, sowie alle organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Bevorzugte Verdünnungsmittel sind Wasser, Alkohole, wie Methanol, Ethanol, n-oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; sowie deren Gemische mit Wasser. Besonders bevorzugte Verdünnungsmittel sind in diesem Fall Wasser oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser.

Als Verdünnungsmittel zur Durchführung der erfindungsgemäßen Verfahren b), d), h), i), l), r) und s) kommen alle inerten organischen Lösungsmittel in Betracht. Hierzu gehören vorzugsweise aromatische Kohlenwasserstoffe, wie beispielsweise Benzol, Toluol oder Xylol; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; organische Säuren, wie Essigsäure; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Besonders bevorzugte Verdünnungsmittel sind Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, Säuren, wie Essigsäure, deren Gemische mit Wasser oder reines Wasser. Weiterhin besonders bevorzugt sind auch Zweiphasengemische, wie beispielsweise Wasser/Toluol.

Als Verdünnungsmittel zur Durchführung der erfindungsgemäßen Verfahren c), e), f), g), o), p), t) und u) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,NN,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Besonders bevorzugte Verdünnungsmittel sind Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether. Weiterhin besonders bevorzugt sind auch Zweiphasengemische, wie beispielsweise Wasser/Toluol.

Als Verdünnungsmittel zur Durchführung der erfindungsgemäßen Verfahren m), n) und q) kommen Wasser, sowie alle organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,NN,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die erfindungsgemäßen Verfahren a), k) und v) werden gegebenenfalls in Gegenwart einer Säure oder einer Base durchgeführt. Als Säuren kommen alle anorganischen und organischen Protonen- wie auch Lewissäuren, sowie auch alle polymeren Säuren infrage. Hierzu gehören beispielsweise Chlorwasserstoff, Bromwasserstoff, Schwefelsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, saure Ionenaustauscher, saure Tonerden und saures Kieselgel. Vorzugsweise kommen Chlorwasserstoff oder Bromwasserstoff infrage. Als Basen kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N.N-Dimethylbenzylamin, Pyridin, N-Methylpiperidin, N-Methylmorpholin, N.N-Dimethylaminopyridin, Diazabicyclooctan (DABCO), Diazabicyclononen (DBN) oder Diazabicycloundecen (DBU). Besonders bevorzugte Basen sind Natrium-methylat, Natrium-ethylat, Kalium-tert.-butylat, Natriumhydroxid, Kaliumhydroxid, Ammoniumhydroxid, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,NN,N-Dimethylaminopyridin, Diazabicyclooctan (DABCO), Diazabicyclononen (DBN) oder Diazabicycloundecen (DBU).

Die erfindungsgemäßen Verfahren b), d), h), i), l), r) und s) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -alkoholate, -acetate, -carbonate oder -hydrogencarbonate, wie beispielsweise Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren c), e), f), g), o), p), t) und u) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -carbonate oder -hydrogencarbonate, wie beispielsweise Natriumhydrid, Natriumamid, Natrium-methylat, Natrium-ethylat, Kalium-tert.butylat, Natriumhydroxid, Kaliumhydroxid, Natriumcarbonat, Kaliumcarbonat, Kaliumhydrogencarbonat oder Natriumhydrogencarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a), k) und v) in einem größeren Bereich variiert werden. Im allgemeinen arbeitet man bei Temperaturen von -20°C bis 100°C, vorzugsweise bei Temperaturen von -10°C bis 80°C.

Die Reaktionstemperaturen können bei der Durchführung der erfindungsgemäßen Verfahren b), d), h), i), l), r und s) in einem größeren Bereich variiert werden. Im allgemeinen arbeitet man bei Temperaturen von 0°C bis 200°C, vorzugsweise bei Temperaturen von 20°C bis 150°C.

Die Reaktionstemperaturen können bei der Durchführung der erfindungsgemäßen Verfahren c), e), f), g), o), p), t) und u) in einem größeren Bereich variiert werden. Im allgemeinen arbeitet man bei Temperaturen von -20°C bis 100°C, vorzugsweise bei Temperaturen von 0°C bis 80°C.

Die erfindungsgemäßen Verfahren a) bis v) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

In einer bevorzugten Verfahrensvariante (A) wird ein Benzofurandionmonooxim der Formel (XI) durch Reaktion mit einem Ethanderivat der Formel (IX) zunächst, wie in Verfahren g) beschrieben, in ein O-Hydroxyethyl-benzofurandionmonooxim der Formel (VII) überführt. Dieses setzt man ohne weitere Reinigung anschließend mit einem Alkoxyamin der Formel (IV) - oder einem Säureadditionskomplex davon - gegebenenfalls in einem Puffersystem, wie beispielsweise Natriumacetat/Essigsäure, wie in Verfahren d) beschrieben, zu einem O-Hydroxyethyl-O'-methyl-benzofurandiondioxim der Formel (II) um, das wiederum ohne weitere Reinigung nach Behandlung mit einer Säure oder einer Base, gemäß Verfahren a), das gewünschte 3-(1-Hydroxyphenyl-1-alkoximinomethyl)dioxazin der Formel (I) liefert.

In einer weiteren bevorzugten Verfahrensvariante (B) wird ein Benzofurandionmonooxim der Formel (XI) durch Reaktion mit einem Ethanderivat der Formel (IX) zunächst, wie in Verfahren g) beschrieben, in ein O-Hydroxyethyl-benzofurandionmonooxim der Formel (VII) überführt. Dieses liefert nach Behandlung mit einer Säure oder einer Base ein Hydroxybenzoyldioxazin der Formel (III), das man anschließend mit einem Alkoxyamin der Formel (IV) - oder einem Säureadditionskomplex davon - zu dem gewünschten 3-(1-Hydroxyphenyl-1-alkoximinomethyl)dioxazin der Formel (I) umsetzt.

In einer dritten bevorzugten Verfahrensvariante (C) wird ein Benzofurandionmonooxim der Formel (XI) durch Reaktion mit einem Alkoxyamin der Formel (IV) - oder einem Säureadditionskomplex davon - zunächst in ein O-Alkyl-benzofurandiondioxim der Formel (VIII) überführt. Dieses setzt man anschließend mit einem Ethanderivat der Formel (IX) zu einem O-Hydroxyethyl-O'-methyl-benzofurandiondioxim der Formel (II) um, das nach Behandlung mit einer Säure oder einer Base das gewünschte 3-(1-Hydroxyphenyl-1-alkoximinomethyl)dioxazin der Formel (I) liefert.

Es ist als äußerst überraschend zu bezeichnen, dass die erfindungsgemäßen Verfahren, insbesondere in ihrer Kombination, in hoher Ausbeute und hoher Reinheit ablaufen. In Chem. Ber. 1902, 1640 ist beispielsweise beschrieben, dass Benzofurandionmonooxime der Formel (XI) sowohl durch Behandlung mit Säuren, wie auch durch Behandlung mit Basen zu Salicylsäurederivaten oder Hydroxyphenylglyoxylsäurederivaten gespalten werden. Somit konnte nicht damit gerechnet werden, dass sich die 3-(1-Hydroxyphenyl-1-alkoximinomethyl)dioxazine in einer nur dreistufigen Reaktion ohne nennenswerte Nebenreaktionen herstellen lassen.

Die erfindungsgemäßen Verfahren weisen eine Reihe von Vorteilen auf. So ermöglichen sie die Herstellung einer Vielzahl von 3-(1-Hydroxyphenyl-1-alkoximinomethyl)dioxazinen in hoher Ausbeute und hoher Reinheit. Günstig ist es auch, dass die als Ausgangsstoffe benötigten Benzofurandionmonooxime in einfacher Weise auch in größeren Mengen zugänglich sind (Beilstein, E (II) 17, 462; Mameli, G. 56, 768).

### Verfahrens- und Herstellungsbeispiele:

### Beispiel 1

### Verfahrensvariante (A)

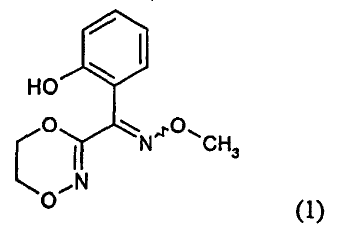

### 1 Stufe

### Verbindung (VII-1):

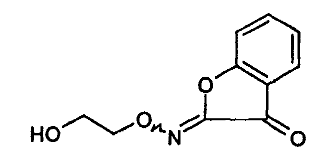

### Verfahren g)

11,8 g (0,0725 Mol) Benzofuran-2,3-dion-2-oxim (XI-1) (Stoermer, Kahlert, B. 35, 1644) werden in 75 ml Dimethylformamid gelöst. Man gibt unter Kühlen 3 g (0,075 Mol) 60 %iges Natriumhydrid (Mineralölsuspension) portionsweise zu und rührt bei einer Temperatur von 25°C ca. eine Stunde bis zum Ende der Gasentwicklung. Anschließend kühlt man auf 0°C, tropft bei dieser Temperatur 9,3 g (0,0744 Mol) 2-Bromethanol zu und rührt noch 24 Stunden bei 25 °C. Man gießt das Reaktionsgemisch auf Wasser, extrahiert mit Essigsäureethylester, trocknet die organische Phase über Natriumsulfat und destilliert das Lösungsmittel im Vakuum ab. Der Rückstand wird aus einer Mischung von 150 ml Toluol und 100 ml Cyclohexan umkristallisiert. Man erhält 11,5 g (64 % der Theorie) Benzofuran-2,3-dion-2-[O-(2-hydroxy-ethyl)-oxim] (VII-1) (Gehalt nach HPLC-Analyse: 83,5 %). Eine aus Toluol umkristallisierte Probe schmilzt bei 110 - 111°C.

### 2 Stufe

### Verbindung (II-1):

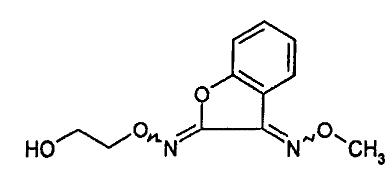

### Verfahren d)

11,5 g rohes Benzofuran-2,3-dion-2-[O-(2-hydroxy-ethyl)-oxim] (VII-1) und 5,15 g (0,0616 Mol) O-Methylhydroxylamin-Hydrochlorid werden in 60 ml Dimethylformamid gelöst und 30 Minuten bei 80°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 14,1 g eines Öles, das 40,5 % Benzofuran-2,3-dion-2-[O-(2-hydroxy-ethyl)-oxim]-3-(O-methyl-oxim) (II-1) und 14 % (5,6-Dihydro-[1,4,2]dioxazin-3-yl)-(2-hydroxy-phenyl)-methanon-O-methyl-oxim (1) enthält.

### 3 Stufe

### Verbindung (1)

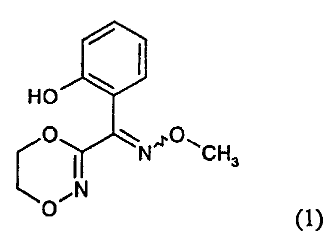

### Verfahren a)

14,1 g des aus der 2. Stufe des Verfahrens A erhaltenen Öles, das die Verbindungen (II-1) und (1) enthält, werden in 200 ml Diethylether gelöst, welcher zuvor bei 0 °C mit Chlorwasserstoffgas gesättigt wurde. Man rührt 30 Minuten ohne weitere Kühlung und gießt die Mischung auf eine auf 0°C gekühlte Natriumhydrogencarbonat-Lösung. Man trennt die organische Phase ab und extrahiert die wässrige Phase noch mehrmals mit Diethylether. Die vereinigten organischen Phasen werden über Natriumsulfat getrocknet und bei vermindertem Druck eingeengt. Der Rückstand wird mit tert.-Butyl-methylether verrührt, wobei das Produkt kristallisiert. Man erhält 5,4 g (28% der Theorie, bezogen auf Benzofuran-2,3-dion-2-oxim) -2-oxim) kristallines (5,6-Dihydro-[1,4,2]dioxazin-3-yl)-(2-hydroxy-phenyl)-methanon-O-methyl-oxim (1) (Gehalt nach HPLC-Analyse: 88,8 %).
¹H-NMR-Spektrum (CDCl₃/TMS): δ = 4,10 (3H); 4,19/4,20/4,21/4,22 (2H); 4,47/4,48/4,49/4,50 (2H); 6,26 (1H); 6,95-7,0 (2H); 7,21/7,23 (1H); 7,31-7,36 (2H) ppm.

### Beispiel 2

### Verfahrensvariante B

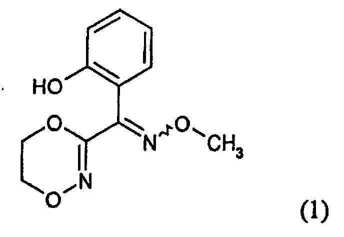

### 1 Stufe

### Verbindung (VII-1):

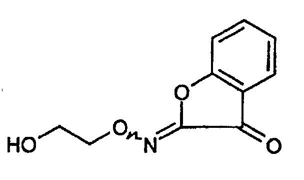

Diese Stufe ist als Verfahren g) in der 1. Stufe der Verfahrensvariante (A) bereits beschrieben worden.

### 2. Stufe

### Verbindung (III-1):

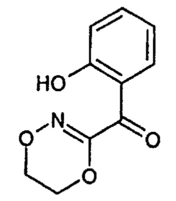

### Verfahren k)

In die Mischung von 19 g (0,0917 Mol) Benzofuran-2,3-dion-2-[O-(2-hydroxyethyl)-oxim] (VII-1) und 439 ml Diethylether leitet man bei 20°C trockenen, gasförmigem Chlorwasserstoff ein, wobei sich das Gemisch auf 35°C erwärmt. Nach 7 Stunden dekantiert man von ungelöstem Material ab und destilliert das Lösungsmittel im Vakuum ab. Der Rückstand wird in Essigsäureethylester aufgenommen und zuerst mit 50 ml Wasser, anschließend mit 20 ml einer gesättigten, wässrigen Natriumhydrogencarbonat-Lösung gewaschen. Man trennt die organische Phase ab, trocknet sie über Natriumsulfat, destilliert das Lösungsmittel im Vakuum ab und erhält 17 g Rohprodukt. Dieses wird mit Dichlormethan an Kieselgel chromatographiert. Man erhält 10 g (51,9 % der Theorie bezogen auf Verbindung (VII-1) (5,6-Dihydro-[1,4,2]dioxazin-3-yl)-(2-hydroxy-phenyl)-methanon (III-1) in einer Reinheit von 98,7 % (HPLC).
¹H-NMR (CDCl₃, TMS): δ = 4,28/4,29/4,30/4,31 (2H); 4,55/4,56/4,57/4,58 (2H); 6,90/6,92/6,93/6,95/6,99/7,02 (2H); 7,50/7,51/7,53/7,54/7,55/7,56 (1H); 8,27/8,29/8,30 (1H); 11,52 (1H) ppm.

### 3. Stufe:

### Verbindung (1):

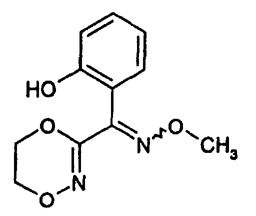

### Verfahren b)

4,4 g (0,021 Mol) (5,6-Dihydro-[1,4,2]dioxazin-3-yl)-(2-hydroxy-phenyl)-methanon (III-1) werden mit 1,87 g (0,022 Mol) O-Methylhydroxylamin-Hydrochlorid in 22 ml Dimethylformamid 2 Stunden auf 100 °C erwärmt. Man gießt das Reaktionsgemisch auf Wasser, extrahiert mit Essigsäureethylester, trocknet die organische Phase über Natriumsulfat und destilliert das Lösungsmittel im Vakuum ab. Man erhält 5,7 g rohes (5,6-Dihydro-[1,4,2]dioxazin-3-yl)-(2-hydroxy-phenyl)-methanon-O-methyloxim (1), das nach HPLC aus 17,5 % E-Isomerem und aus 38 % Z-Isomerem besteht. Dieses Rohprodukt wird in einer Mischung von Petrolether/tert.-Butylmethylether (1:1) an Kieselgel chromatographiert. Man erhält 1,55 g Z-(5,6-Dihydro-[1,4,2]dioxazin-3-yl)-(2-hydroxy-phenyl)-methanon-O-methyl-oxim (1) in einer Reinheit von 91,5 % (HPLC) = 28,27 % der Theorie.
¹H-NMR-Spektrum (CDCl₃/TMS): δ = 4,08 (3H); 4,30/4,32/4,33 (2H); 4,53/4,54/4,55 (2H); 6,89/6,92/6,94/6,98/7,01 (2H); 7,28/7,31/7,33/7,34/7,35/7,37 (2H); 10,11 (1H)ppm.

Ferner erhält man 0,9 g E-3(5,6-Dihydro-[1,4,2]dioxazin-3-yl)-(2-hydroxy-phenyl)-methanon-O-methyl-oxim (1) in einer Reinheit von 93,7 % (HPLC) = 16,8 % der Theorie.

¹H-NMR-Spektrum (CDCl₃/TMS): δ = 4,09 (3H); 4,18/4,19/4,20/4,21 (2H); 4,47/4,48/4,50 (2H); 6,28 (1H: OH); 6,94/6,95/6,97(2H); 7,20-7,36 (2H) ppm.

### Beispiel 3:

### Verfahrensvariante C

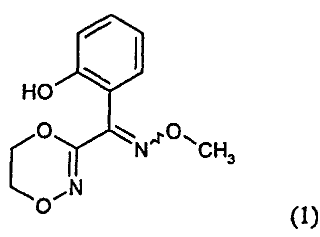

### 1. Stufe

### Verbindung (VIII-1):

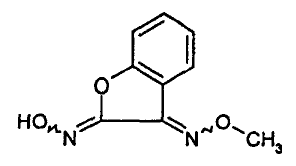

### Verfahren h)

3,26 g (0,02 Mol) Benzofuran-2,3-dion-2-oxim (XI-1) werden in 20 ml Dimethylformamid mit 3,36 g (0,04 Mol) O-Methylhydroxylamin Hydrochlorid 20 Minuten bei 20°C und dann 45 Minuten bei 80°C gerührt. Man gießt in eine Mischung aus wässriger Natriumhydrogencarbonat-Lösung und Essigsäureethylester. Man trennt die organische Phase ab, trocknet sie über Natriumsulfat und destilliert das Lösungsmittel bei vermindertem Druck ab. Der Rückstand wird mit Diethylether verrührt. Man erhält 1,4 g kristallines Benzofüran-2,3-dion-3-(O-methyl-oxim)-2-oxim (VIII-1), das nach HPLC-Analyse 70,7 % vom Stereoisomeren A und 8,5 % vom Stereoisomerem B enthält.
¹H-NMR-Spektrum (CDCl₃/TMS): δ = 4,09 (3H, Isomer B); 4,11 (3H, Isomer A); 7,21-7,35 (2H); 7,51-7,65 (1H, Isomer A und 2H, Isomer B); 8,02/8,04/8,05 (1H, Isomer A); 11,35 (1H, Isomer A); 11,74 (1H, Isomer B) ppm.

### 2- Stufe

### Verbindung (II-1):

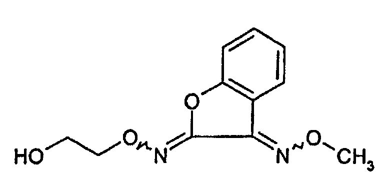

### Verfahren e)

4,49 g (0,0234 Mol) Benzofuran-2,3-dion-3-(O-methyl-oxim)-2-oxim (VIII-1) werden in 25 ml Dimethylformamid gelöst. Zur Lösung gibt man 1 g (0,025 Mol) 60 %iges Natriumhydrid und rührt eine Stunde bei Raumtemperatur. Man gibt 3,1 g (0,0248 Mol) 2-Bromethanol zu und rührt 12 Stunden bei 25°C. Nach Zugabe von weiteren 0,5 g Natriummethylat und 1,22 g 2-Bromethanol rührt man 2 Stunden bei Raumtemperatur. Man gibt nochmals 0,5 g Natriummethylat und 1,22 g 2-Bromethanol zu und rührt weitere 2 Stunden bei Raumtemperatur. Man gießt das Reaktionsgemisch auf Wasser, extrahiert mit Essigsäureethylester, wäscht die organische Phase mit 3 mal 20 ml 2 N Natronlauge. Man destilliert das Lösungsmittel im Vakuum ab und chromatographiert den Rückstand in Diethylether/Petrolether (1:1) an Kieselgel. Man destilliert das Laufmittel im Vakuum ab und erhält 2,26 g (39,6% der Theorie) Benzofüran-2,3-dion-2-[O-(2-hydroxy-ethyl)-oxim]-3-(O-methyl-oxim) (II-1), das nach HPLC aus 84,29 % Stereoisomer A und aus 12,58 % Stereoisomer B besteht.
1H-NMR-Spektrum (CDCl3/TMS):
δ = 3,95-4,03 (2H); 4,20 (3H, Isomer B); 4,21 (3H, Isomer A); 4,37-4,40 (2H); 7,14-7,21 (2H); 7,40-7,49 (1H), 7,63/7,64/7,66 (1H, Isomer B); 8,04/8,06/8,07 (1H, Isomer A) ppm.

### 3. Stufe

### Verbindung (1):

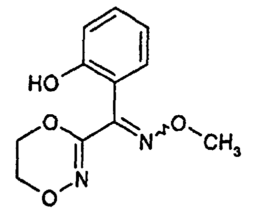

### Verfahren a)

2 g Benzofuran-2,3-dion-2-[O-(2-hydroxy-ethyl)-oxim]-3-(O-methyl-oxim) (II-1) (Gehalt nach HPLC-Analyse 96,88 %, 0,0082 Mol), welches nach Verfahren e) hergestellt wurde, werden in 50 ml Diethylether gelöst, welcher zuvor bei 0°C mit mit Chlorwasserstoffgas gesättigt wurde. Man rührt 30 Minuten ohne weitere Kühlung, destilliert anschließend das Lösungsmittel im Vakuum ab und versetzt den Rückstand wieder mit Diethylether. Ein Teil des Produktes kristallisiert aus und wird abfiltriert. Die Mutterlauge wird im Vakuum eingeengt und der Rückstand in 25 ml in bei 0°C mit Chlorwasserstoffgas gesättigtem Diethylether gelöst. Man rührt wiederum 30 Minuten ohne weitere Kühlung, destilliert das Lösungsmittel im Vakuum ab und versetzt den Rückstand wiederum mit Diethylether. Es kristallisiert eine weiterer Teil des Produktes, der ebenfalls abfiltriert wird. Insgesamt erhält man 1,37 g (69 % der Theorie) (5,6-Dihydro-[1,4,2]dioxazin-3-yl)-(2-hydroxy-phenyl)-methanon-O-methyl-oxim (1) (Gehalt nach HPLC-Analyse: 97,59 %).
¹H-NMR-Spektrum (CDCl₃/TMS):
δ= 4,09 (3H); 4,18/4,19/4,20/4,21 (2H); 4,47/4,48/4,50 (2H); 6,94/6,95/6,97(2H); 7,20-7,36 (2H)ppm.

### Beispiel 4:

### Verfahrensvariante (D)

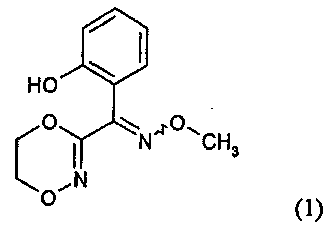

### 1. Stufe:

### Verbindung (XIV-1):

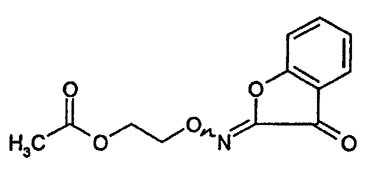

### Verfahren o)

1,63 g (0,01 Mol) Benzofuran-2,3-dion-2-oxim (XI-1) werden in 5 ml N-Methyl-2-pyrrolidinon mit 1,14 g (0,0108 Mol) Natriumcarbonat 30 Minuten bei 20°C gerührt. Nach Zugabe von 1,7 g (0,0102 Mol) Essigsäure-2-bromethylester rührt man 2 Stunden bei 70°C. Man gießt die Mischung auf 30 ml Wasser und filtriert das Produkt ab. Man trocknet es und erhält 1,57 g (63 % der Theorie) kristallinen Essigsäure-2-(3-oxo-3H-benzofuran-2-ylidenaminooxy)-ethylester (XIV- 1).
¹H-NMR-Spektrum (CDCl₃/TMS): δ = 2,11 (3H); 4,43/4,44/4,45/4,46/4,47 (2H); 4,52/4,54/4,55/4,57 (2H); 7,28/7,30/7,33 (2H); 7,72/7,74/7,76/7,78/7,80 (2H) ppm.

### 2. Stufe:

### Verbindung (XIII-1):

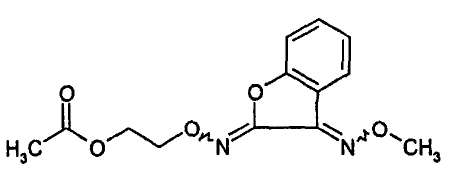

### Verfahren s)

10 g (0,04 Mol) Essigsäure-2-(3-oxo-3H-benzofuran-2-ylidenaminooxy)-ethylester (XIV-1) und 4,18 g (0,05 Mol) O-Methylhydroxylamin-Hydrochlorid werden in 40 ml N-Methyl-2-pyrrolidihon gelöst und 1 Stunde bei 80°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 10,6 g Rohprodukt, welches mit tert.-Butylmethylether/-Petrolether (1:1) an Kieselgel chromatographiert wird. Man erhält 6,9 g (59,1 % der Theorie) Essigsäure-2-(3-methoxyimino-3H-benzoruran-2-ylidenaminooxy)-ethylester (XIII-1) mit einem Gehalt von 82,15 % Isomer A und 13,38 % Isomer B (HPLC).
¹H-NMR-Spektrum (CDCl₃/TMS): δ = 2,10 (3H); 4,21 (3H, Isomer B); 4,22 (3H, Isomer A); 4,41-4,48 (4H); 7,15/7,17/7,20 (2H); 7,44-7,50 (1H); 7,63-7,66 (1H, Isomer B); 8,05-8,09 (1H, Isomer A) ppm.

### 3. Stufe:

### Verbindung (II-1)

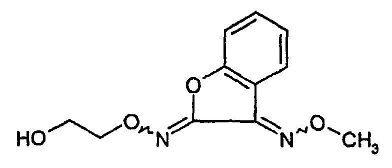

### Verfahren m)

2 g (0,00718 Mol) Essigsäure-2-(3 -methoxyimino-3H-benzofuran-2-ylidenaminooxy)-ethylester (XIII-1) werden in 8 ml Dimethylformamid bei 20°C mit 7,2 ml (0,0144 Mol) 2N Natronlauge versetzt und 16 Stunden bei 20°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 1,7 g (67,6 % der Theorie) Benzofüran-2,3-dion-2-[O-(2-hydroxy-ethyl)-oxim]-3-(O-methyl-oxim) (II-1), welches nach HPLC 56,8% des Stereoisomeren A und 10,7 % des Stereoisomeren B enthält.

Daten aus GC/MS-Analyse (die Substanz wurde vor der Analyse mit N-Methyl-N-trimethylsilyltrifluoracetamid silyliert):
Stereoisomer A:
   Retentionsindex = 2062
   M⁺ = 309, 308, 293, 249, 233, 192, 176, 145, 132, 89, 73, 45, 26.
Stereoisomer B:
   Retentionsindex = 2000
   M⁺ = 309, 308, 293, 249, 218, 192, 176, 145, 132, 90, 73, 45.

### 4. Stufe:

### Verbindung (1)

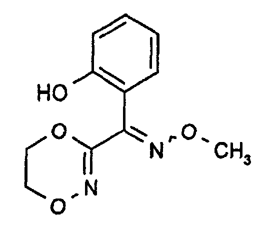

Diese Stufe ist als Verfahren a) in der 3. Stufe der Verfahrensvariante (A) und in der 3. Stufe der Verfahrensvariante (C) bereits beschrieben worden.

### Weitere Beispiele zu einzelnen Verfahren

### Beispiel 5

### Verbindung (1):

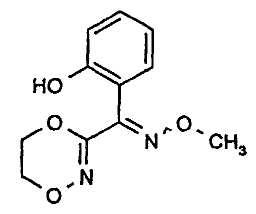

### Verfahren c)

1,2 g (0,0054 Mol) Z-(5,6-Dihydro-[1,4,2]dioxazin-3-yl)-(2-hydroxy-phenyl)-methanon-oxim (V-1) werden in 5 ml Dimethylformamid mit 0,66 g (0,0062 Mol) Natriumcarbonat zunächst 30 Minuten bei 20°C gerührt. Dann gibt man 0,83 g (0,00658 Mol) Dimethylsulfat zu und rührt weitere 16 Stunden bei 20°C. Man stellt das Reaktionsgemisch mit 2N wässriger Salzsäure schwach sauer und extrahiert mit Essigsäureethylester. Man trocknet die organische Phase über Natriumsulfat und destilliert das Lösungsmittel im Vakuum ab. Man erhält 0,8 g (50,35 % der Theorie) Z-(5,6-Dihydro-[1,4,2]dioxazin-3-yl)-(2-hydroxy-phenyl)-methanon-O-niethyl-oxim (1) in einer Reinheit von 80,3 % (HPLC).
¹H-NMR-Spektrum (CDCl₃/TMS): δ = 4,08 (3H); 4,30/4,32/4,33 (2H); 4,53/4,54/4,55 (2H); 6,89/6,92/6,94/6,98/7,01 (2H); 7,28/7,31/7,33/7,34/7,35/7,37 (2H); 10,11 (1H) ppm.

### Beispiel 6

### Verbindung (II-1):

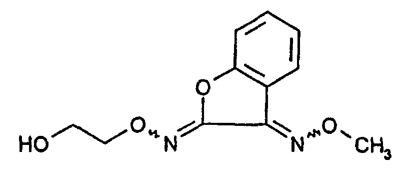

### Verfahren e)

3,19 g (0,0166 Mol) Benzofuran-2,3-dion-3-(O-methyl-oxim)-2-oxim (VIII-1) (Gemisch aus 2 Stereoisomeren A:B = 13:86) werden in 20 ml Methanol suspendiert und tropfenweise bei 20°C mit 8,3 g einer 2 molaren Natriummethylatlösung versetzt. Nachdem die Mischung homogen geworden ist, destilliert man das Methanol im Vakuum ab und trocknet den kristallinen Rückstand 12 Stunden im Exsiccator. Man erhält 3,55 g des Natriumsalzes von Benzofuran-2,3-dion-3-(O-methyl-oxim)-2-oxim (VIII-1). Dieses suspendiert man bei 20°C in 16 ml N-Methyl-2-pyrrolidinon und gibt 2,1 g (0,0168 Mol) 2-Bromethanol zu. Man rührt 48 Stunden bei 20 °C. Man gießt das Reaktionsgemisch auf Wasser, extrahiert mit Essigsäureethylester und wäscht die organische Phase mit 3 mal 20 ml 2 N Natronlauge. Man destilliert das Lösungsmittel im Vakuum ab und chromatographiert den Rückstand mit Diethylether/Petrolether (1:1) an Kieselgel. Man destilliert das Laufmittel im Vakuum ab und erhält 2,91 g (73,8 % der Theorie) Benzofuran-2,3-dion-2-[O-(2-hydroxy-ethyl)-oxim]-3-(O-methyl-oxim) (II-1), bestehend aus 88,6 % Stereoisomer B, 7,7 % Stereoisomer A und 3,2 % Stereoisomer C (HPLC).
¹H-NMR-Spektrum (CDCl₃/TMS): δ = 3,43 (1H, breit); 4,0-4,03 (2H); 4,20 (3H); 4,38-4,41 (2H); 7,14-7,21 (2H); 7,40/7,42/7,43/7,445 (1H); 7,63/7,64/7,66 (1H) ppm.

### Beispiel 7

### Verbindung (II-1):

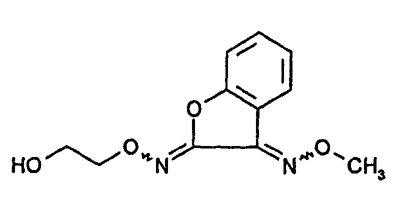

### Verfahren f)

0,44 g (0,002 Mol) Benzofuran-2,3-dion-2-[O-(2-hydroxy-ethyl)-oxim]-3-oxim (X-1) werden bei 20°C in 5 ml Dimethylformamid 20 Minuten mit 0,08 g (0,002 Mol) 60 %igem Natriumhydrid gerührt. Nach beendeter Gasentwicklung gibt man 0,28 g (0,002 Mol) Methyljodid zu und rührt 16 Stunden bei 20°C. Man gießt das Reaktionsgemisch auf Wasser, extrahiert mit Essigsäureethylester, trocknet die organische Phase über Natriumsulfat, destilliert das Lösungsmittel im Vakuum ab und erhält 0,5 g (35 % der Theorie) Rohprodukt. Dieses enthält gemäß der HPLC-Analyse 23 % des Stereoisomeren A und 10,1% des Stereoisomeren B von Benzofuran-2,3-dion-2-[O-(2-hydroxy-ethyl)-oxim]-3-(O-methyl-oxim) (II-1).

Daten aus GC/MS-Analyse (die Substanz wurde vor der Analyse mit N-Methyl-N-trimethylsilyltrifluoracetamid silyliert):
Retentionsindex = 2053 (Isomer A)
M⁺ = 309, 308, 293, 249, 233, 192, 176, 145, 132, 89, 73, 45, 26.
Retentionsindex = 1997 (Isomer B)
M⁺ = 309, 308, 293, 249, 218, 192, 176, 145, 132, 90, 73, 45.

Femer enthält das Rohprodukt nach 20,5 % (HPLC) N-[2-(2-Hydroxy-ethoxyimino)-benzofuran-3-ylidene]-N-methylamin-N-oxid.

GC/MS-Analyse (silylierte Probe):
Retentionsindex = 2234
M⁺ = 310, 308, 233, 192, 175, 159, 132, 102, 73, 45, 26).

### Beispiel 8

### Verbindung (X-1):

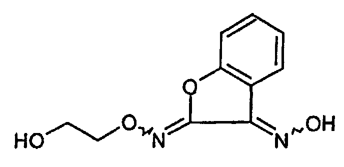

### Verfahren i)

10,1 g (0,05 Mol) Benzofuran-2,3-dion-2-[O-(2-hydroxy-ethyl)-oxim] (VII-1) werden in 50 ml N-Methyl-2-pyrrolidinon mit 3,5 g Hydroxylamin Hydrochlorid 2 Stunden bei 80 °C gerührt. Man gießt das Reaktionsgemisch auf Wasser, extrahiert mit Essigsäure-ethylester, trocknet die organische Phase über Natriumsulfat und destilliert das Lösungsmittel im Vakuum ab. Dann chromatographiert man den Rückstand in tert.-Butyltert.-Butyl-methylether/Petrolether (1:1) an Kieselgel. Man destilliert das Laufmittel im Vakuum ab und erhält 4,2 g (29,6 % der Theorie) Benzofüran-2,3-dion-2-[O-(2-hydroxy-ethyl)-oxim]-3-oxim, bestehend aus 62,4 % Isomer A und 15,8 % Isomer B (HPLC).
¹H-NMR-Spektrum (DMSO-d₆/TMS): δ = 3,64-3,71 (2H); 4,10-4,26 (2H); 4,78-4,87 (1H); 7,2-7,3 (1H); 7,3-7,4 (1H); 7-5-7,7 (1H); 8,11-8,14 (1H); 12,82 (1H, Isomer A); 12,91 (1H, Isomer B) ppm.

### Beispiel 9

### Verbindung (V-1):

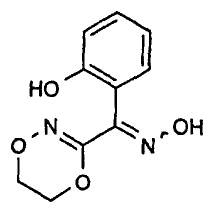

### Verfahren 1)

4,14 g (0,02 Mol) (5,6-Dihydro-[1,4,2]dioxazin-3-yl)-(2-hydroxy-phenyl)-methanon (III-1) werden in 20 ml Dimethylformamid 2 Stunden mit 2,1 g (0,03 Mol) Hydroxylamin Hydrochlorid bei 80°C gerührt. Das Reaktionsgemisch wird auf Wasser gegossen, mit Essigsäureethylester extrahiert, die organische Phase über Natriumsulfat getrocknet und im Vakuum eingeengt. Man erhält 4,7 g Rohprodukt, bestehend aus 15 % E- und 57,5 % Z-Isomer (HPLC). Dieses wird mit einer Mischung aus. Diethylether und Petrolether (1:1) an Kieselgel chromatographiert. Man erhält 2,7 g (60,8 % der Theorie) (5,6-Dihydro-[1,4,2]dioxazin-3-yl)-(2-hydroxy-phenyl)-methanon-oxim (V-1) (Gehalt nach HPLC-Analyse: 93,4 %).
¹H-NMR-Spektrum (DMSO-d₆/TMS): δ = 4,19/4,20/4,21 (2H); 4,45/4,46/4,47 (2H); 6,89-6,92 (2H); 7,22-7,32 (1H); 7,33-7,40 (1H); 10,30 (1H); 12,16 (1H) ppm.

### Beispiel 10

### Verbindung (VII-1)

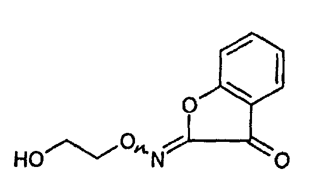

### Verfahren n)

2,07 g (0,01 Mol) Benzofuran-2,3-dione-2-{O-[2-(tetrahydropyran-2-yloxy)-ethyl]-oxim} (XIV-2) werden in 12 ml Methanol gelöst und mit 100 mg saurem lonenaustauscherharz 16 Stunden bei Raumtemperatur gerührt. Man versetzt das Reaktionsgemisch mit 40 ml Methanol, erwärmt bis die Kristalle gelöst sind und filtriert vom sauren Ionenaustauscherharz ab. Das Filtrat wird eingeengt und der Rückstand aus 10 ml Toluol umkristallisiert. Man erhält 1,69 g (81,5 % der Theorie) kristallines Benzofuran-2,3-dion-2-[O-(2-hydroxy-ethyl)-oxim] (VII-1) vom Schmelzpunkt 110-111°C.
¹H-NMR-Spektrum (CDCl₃/TMS): δ= 2,33 (1H, breit), 4,00-4,03 (2H); 4,47-4,50 (2H); 7,27-7,32 (2H); 7,70-7,78 (2H) ppm.

### Beispiel 11

### Verbindung (XIV-2):

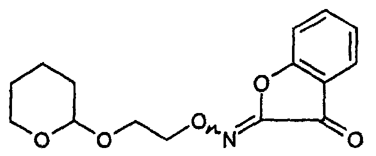

### Verfahren o)

5 g (0,03 Mol) Benzofuran-2,3-dion-2-oxim (XI-1) werden in 30 ml Methanol gelöst und bei 20°C tropfenweise mit 15 ml 2 molarer Natriummethylatlösung in Methanol versetzt. Man destilliert das Lösungsmittel im Vakuum ab. Der kristalline Rückstand wird in 30 ml N-Methyl-2-pyrrolidinon gelöst und bei 20°C mit 6,27 g (0,03 Mol) 2-(2-Bromethoxy)-tetrahydropyran versetzt. Man rührt 16 Stunden bei 20°C, gießt das Reaktionsgemisch auf 100 ml Wasser und extrahiert zweimal mit je 100 ml Dichlormethan. Nach dem Abdestillieren des Lösungsmittels im Vakuum chromatographiert man den Rückstand in einer Mischung aus Diethylether/Dichlormethan/-Petrolether (1:1:2) an Kieselgel. Man erhält 5,55 g (62,1% der Theorie) Benzofuran-2,3-dion-2-{O-[2-(tetrahydropyran-2-yloxy)-ethyl-oxim} (XIV-2).
¹H-NMR-Spektrum (CDCl₃/TMS): δ = 1,50-1,86 (6H); 3,49-3,53 (1H); 3,81-3,88 (2H); 4,02-4,09 (1H); 4,52-4,55 (2H); 4,66-4,69 (1H); 7,26-7,31 (2H); 7,69-7,80 (2H) ppm.

### Beispiel 12

### Verbindung (VIII-1)

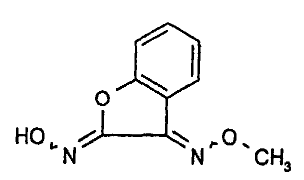

### Verfahren p)

Zu einer Suspension von 2,4 g (0,06 Mol) 60%igem Natriumhydrid in 25 ml Dimethylformamid tropft man eine Lösung von 11 g (0,0617 Mol) Benzofurandion-2,3-dioxim in 50 ml Dimethylformamid und rührt eine Stunde bei 20°C. Dann tropft man 7,55 g (0,06 Mol) Dimethylsulfat zu und rührt weitere 16 Stunden bei 20°C. Man gießt das Reaktionsgemisch auf Wasser und extrahiert mit Essigsäureethylester, trocknet die organische Phase über Natriumsulfat und engt im Vakuum ein. Der Rückstand wird mit Hexan/Aceton (7:3) an Kieselgel chromatografiert. Nach dem Verrühren des Rückstandes mit Diethylether erhält man 0,7 g Benzofuran-2,3-dion-3-(O-methyl-oxim)-2-oxim (VIII-1) als Stereoisomerengemisch, bestehend aus 90,6 % Isomer A und 9 % Isomer B (HPLC).

### Beispiel 13

### Verbindung (X-1):

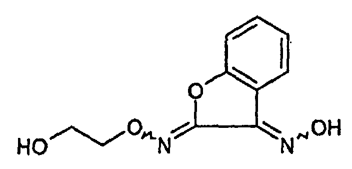

### Verfahren q)

5,28 g (0,02 Mol) Essigsäure-2-(3-hydroxyimino-3H-benzofuran-2-ylideneaminooxy)-ethylester (XVI-1) werden in 20 ml Dimethylformamid mit 4,24 g (0,048 Mol) 45 %iger Natronlauge 3 Stunden bei 20°C gerührt. Man säuert mit 2N Salzsäure an, extrahiert mit Essigsäureethylester, trocknet die organische Phase über Natriumsulfat und destilliert das Lösungsmittel im Vakuum ab. Das Rohprodukt enthält nach HPLC-Analyse 23,4 % Stereoisomer A und 4,1% des Stereoisomer B von Benzofuran-2,3-dion-2-[O-(2-hydroxy-ethyl)-oxim]-3-oxim (X-1). Nach dem Verrühren mit Diethylether erhält man 1,4 g (28,5 % der Theorie) kristallines Benzofuran-2,3-dion-2-[O-(2-hydroxy-ethyl)-oxim]-3-oxim (X-1), bestehend aus 84,7 % Stereoisomer A und 6 % Stereoisomer B (HPLC).
¹H-NMR-Spektrum (DMSO-d₆/TMS): δ = 3,64-3,71 (2H); 4,10-4,26 (2H); 4,78-4,87 (1H); 7,2-7,3 (1H); 7,3-7,4 (1H); 7-5-7,7 (1H); 8,11-8,14 (1H); 12,82 (1H, Stereoisomer A); 12,91 (1H, Stereoisomer B) ppm.

### Beispiel 14

### Verbindung (XVI-1):

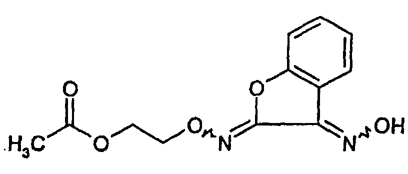

### Verfahren r)

5 g (0,02 Mol) Essigsäure-2-(3-oxo-3H-benzofuran-2-ylidenaminooxy)-ethylester (XIV-1) und 1,74 g (0,025 Mol) Hydroxylamin-Hydrochlorid werden in 20 ml Dimethylformamid gelöst und 2 Stunden bei 100°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Der Rückstand wird mit tert.-Butylmethylether/Petrolether (1:1) an Kieselgel chromatographiert. Man erhält 2,7 g (38,5 % der Theorie) Essigsäure-2-(3-hydroxyimino-3H-benzofuran-2-ylideneaminooxy)-ethylester (XVI-1), bestehend aus 64,23 % Stereoisomer A und 14,9 % Stereoisomer B (HPLC).
¹H-NMR-Spektrum (CDCl₃/TMS): δ = 2,11 (3H, Stereoisomer A); 2,12 (3H, Stereoisomer B); 4,43-4,46 (4H); 7,19-7,23 (2H); 7,46-7,52 (1H); 7,65-7,8 (1H, Stereoisomer B); 8,16/8,17/8,19 (1H, Stereoisomer A); 8,9 (1H) ppm.

### Beispiel 15

### Verbindung (XIII-1)

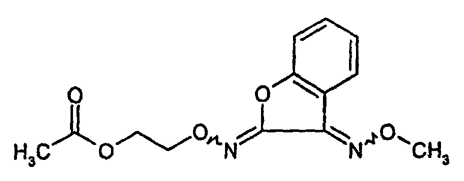

### Verfahren s)

10 g (0,04 Mol) Essigsäure-2-(3-oxo-3H-benzofuran-2-ylidenaminooxy)-ethylester (XIV-1) und 4,18 g (0,05 Mol) O-Methylhydroxylamin-Hydrochlorid werden in 40 ml N-Methyl-2-pyrrolidinon gelöst und 1 Stunde bei 80°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 10,6 g rohen Essigsäure-2-(3-methoxyimino-3H-benzofuran-2-ylidenaminooxy)-ethylester (XIII-1), welcher mit einer Mischung aus tert.-Butylmethylether und Petrolether (1:1) an Kieselgel chromatographiert wird. Man erhält 6,9 g (59,1 % der Theorie) Essigsäure-2-(3-methoxyimino-3H-benzofuran-2-ylidenaminooxy)-ethylester (XIII-1) mit einem Gehalt von 82,15 % Stereoisomer A und 13,38 % Stereoisomer B.
¹H-NMR-Spektrum (CDCl₃/TMS): δ = 2,10 (3H); 4,21(3H, Stereoisomer B); 4,22(3H, Stereoisomer A); 4,41-4,48 (4H); 7,15/7,17/7,20 (2H); 7,44-7,50 (1H); 8,05-8,09 (1H) ppm.

### Beispiel 16

### Verbindung (XIII-1):

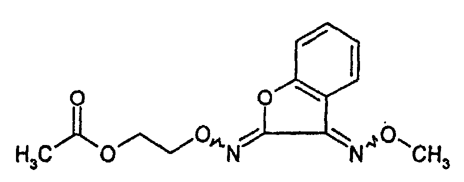

### Verfahren t)

0,26 g (0,001 Mol) Essigsäure-2-(3-hydroxyimino-3H-benzofuran-2-ylidenaminooxy)-ethylester (XVI-1) werden in 2 ml Dimethylformamid gelöst. Man gibt 0,04 g (0,001 Mol) 60 %iges Natriumhydrid bei 20°C dazu und rührt bis zum Ende der Gasentwicklung. Dann gibt man 0,05 g (0,0005 Mol) Natriumcarbonat und 0,13 g (0,001 Mol) Dimethylsulfat zum Reaktionsgemisch und lässt es 2 Tage bei 20°C stehen. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 0,24 g (42,5 % der Theorie) rohen Essigsäure-2-(3-methoxyimino-3H-benzofuran-2-ylidenaminooxy)-ethylester (XIII-1), der nach HPLC-Analyse 33,9 % Stereoisomer A und 15,4 % Stereoisomer B enthält.

Daten aus der GC/MS-Analyse:
Retentionsindex = 2097 (Stereoisomer A)
M⁺ = 279, 278, 218, 187, 160, 144, 130, 87, 75, 43, 26.
Retentionsindex = 2036 (Stereoisomer B)
M⁺=279, 278, 218, 187, 160, 144, 130, 87, 63, 43, 26.

### Beispiel 17

### Verbindung (XIII-1):

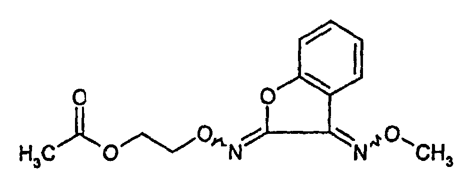

### Verfahren u)

1,92 g (0,01 Mol) Benzofuran-2,3-dion-3-(O-methyl-oxim)-2-oxim (VIII-1) werden in 10 ml Dimethylformamid bei 20°C gelöst. Zu dieser Lösung gibt man 0,4 g (0,01 Mol) 60 %iges Natriumhydrid und rührt 1 Stunde bei 20°C. Nun gibt man 1,67 g (0,01 Mol) Essigsäure-2-bromethylester zu und rührt 16 Stunden bei 20°C. Das Reaktionsgemisch wird auf Wasser gegossen und mit Essigsäureethylester extrahiert. Die organische Phase wird über Natriumsulfat getrocknet und im Vakuum eingeengt. Man erhält 2,3 g (52,6 % der Theorie) Essigsäure-2-(3-methoxyimino-3H-benzofuran-2-ylidenaminooxy)-ethylester (XIII-1), bestehend aus 51,17 % Stereoisomer A und 12,49 % Stereoisomer B (HPLC).

Daten aus GC/MS-Analyse (die Substanz wurde vor der Analyse mit N-Methyl-N-trimethylsilyltrifluoracetamid silyliert):
Retentionsindex = 2097 (Stereoisomer A)
M⁺ = 279, 278, 218, 187, 160, 144, 130, 87, 75, 43, 26.
Retentionsindex = 2035 (Stereoisomer B)
M⁺ = 279, 278, 218, 187, 160, 144, 130, 87, 75, 43, 26.

### Beispiel 18

### Verbindung (V-1):

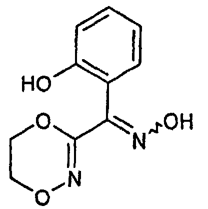

### Verfahren v)

1,11 g (0,005 Mol) Benzofüran-2,3-dion-2-[O-(2-hydroxy-ethyl)-oxim]-3-oxim (X-1) werden in 20 ml Diethylether gelöst, welcher zuvor bei 0°C mit Chlorwasserstoffgas gesättigt wurde. Man rührt 3 Stunden ohne weitere Kühlung und gießt die Mischung auf eine auf 0°C gekühlte Natriumhydrogencarbonat-Lösung. Man trennt die organische Phase ab und extrahiert die wässrige Phase noch mehrmals mit Essigsäureethylester. Die vereinigten organischen Phasen werden über Natriumsulfat getrocknet und bei vermindertem Druck eingeengt. Man erhält 0,8 g (29,9 % der Theorie) rohes (5,6-Dihydro-[1,4,2]dioxazin-3-yl)-(2-hydroxy-phenyl)-methanon-oxim (V-1), bestehend aus 19,7 % E-Isomer und 21,8 % Z-Isomer (HPLC).

Daten aus GC/MS-Analyse (die Substanz wurde vor der Analyse mit N-Methyl-N-trimethylsilyltrifluoracetamid silyliert):
Retentionsindex = 1980 (E-Isomer)
M⁺ = 368, 351, 307, 292, 250, 235, 203, 176, 147, 117, 100, 73, 45.
Retentionsindex = 2036 (Z-Isomer)
M⁺=367, 351, 306, 292, 250, 235, 203, 176, 147, 117, 100, 73, 45.

## Patentansprüche

1. Verbindungen der Formel (VIII) 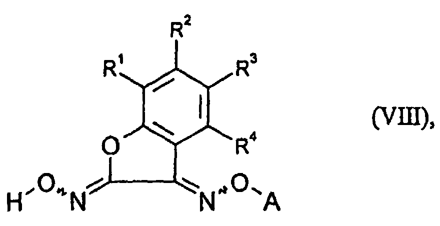 in welcher
A für Alkyl steht und
R¹, R², R³ und R⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen.

2. Verbindungen der Formel (VIII), gemäß Anspruch 1,
in welcher
A für Methyl, Ethyl, n-, oder i-Propyl steht,
R¹, R², R³ und R⁴, gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen, steht.

3. Verbindungen der Formel (VIII), gemäß Anspruch 1, in welcher
A für Methyl oder Ethyl steht,
R¹, R², R³ und R⁴ gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen.

4. Verfahren zur Herstellung von Verbindungen der Formel (VIII) wie in Anspruch 1 definiert, **dadurch gekennzeichnet, dass** man
h) Benzofurandionmonooxime der Formel 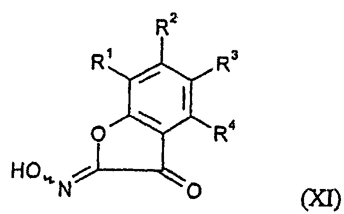 mit einem Alkoxyamin der Formel (IV),
A-O-NH₂ (IV)
gegebenenfalls in Gegenwart eines Verdünnungsmittels und gegebenenfalls in Gegenwart eines Säureakzeptors, umsetzt, oder dass man
p) Benzofurandiondioxime der Formel 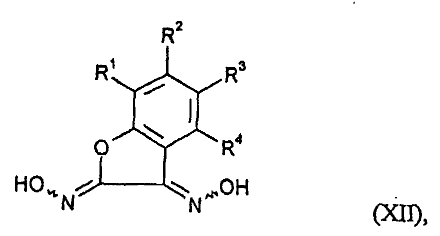 in welcher
R¹, R², R³ und R⁴ die in Anspruch 1 angegebenen Bedeutungen haben,
mit einem Alkylierungsmittel der Formel (VI),
A-X (VI)
gegebenenfalls in Gegenwart eines Verdünnungsmittels, und gegebenenfalls in Gegenwart einer Base, umsetzt.

## Claims

1. Compounds of the formula (VIII) 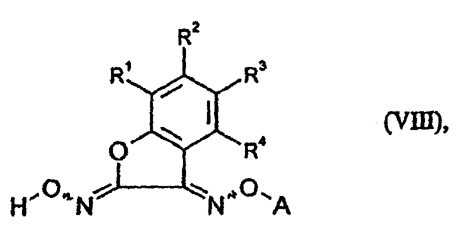 in which
A represents alkyl,
R¹, R², R³ and R⁴ are identical or different and each represent independently of one another hydrogen, halogen, cyano, nitro, respectively optionally halogen-substituted alkyl, alkoxy, alkylthio, alkylsulphinyl or alkylsulphonyl.

2. Compounds of the formula (VIII) according to Claim 1,
in which
A represents methyl, ethyl, n- or i-propyl,
R¹, R², R³ and R⁴ are identical or different and each represent independently of one another hydrogen, halogen, cyano or nitro, or represent alkyl, alkoxy, alkylthio, alkylsulphinyl or alkylsulphonyl, each of which is optionally substituted by 1 to 5 halogen atoms and each of which has 1 to 6 carbon atoms.

3. Compounds of the formula (VIII) according to Claim 1,
in which
A represents methyl or ethyl,
R¹, R², R³ and R⁴ are identical or different and each represent independently of one another hydrogen, fluorine, chlorine, bromine, cyano, nitro, methyl, ethyl, n- or i-propyl, n-, i-, s- or t-butyl, methoxy, ethoxy, n- or i-propoxy, methylthio, ethylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl.

4. Process for preparing compounds of the formula (VIII) as defined in Claim 1, **characterized in that**
h) benzofurandione monooximes of the formula 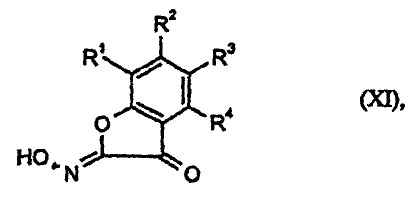 are reacted with an alkoxyamine of the formula (IV),
A-O-NH₂ (IV)
if appropriate in the presence of a diluent and, if appropriate, in the presence of an acid acceptor, or
p) benzofurandione dioximes of the formula 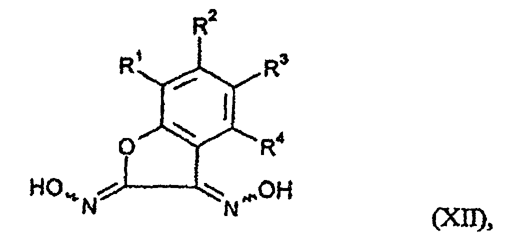 in which
R¹, R², R³ and R⁴ are each as defined in Claim 1,
are reacted with an alkylating agent of the formula (VI)
A-X (VI)
if appropriate in the presence of a diluent and,
if appropriate, in the presence of a base.

## Revendications

1. Composés de formule (VIII) 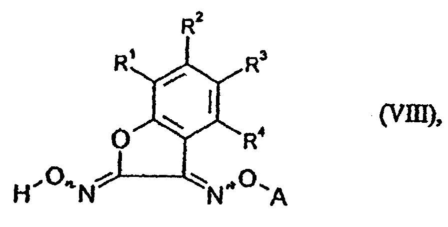 dans laquelle
A est un reste alkyle et
R¹, R², R³ et R⁴ sont identiques ou différents et représentent chacun, indépendamment l'un de l'autre, l'hydrogène, un halogène, un groupe cyano, nitro, un reste alkyle, alkoxy, alkylthio, alkylsulfinyle ou alkylsulfonyle dont chacun est éventuellement substitué par un halogène.

2. Composés de formule (VIII) suivant la revendication 1,
formule dans laquelle
A est un reste méthyle, éthyle, n-propyle ou isopropyle,
R¹, R², R³ et R⁴ sont identiques ou différents et représentent chacun, indépendamment l'un de l'autre, l'hydrogène, un halogène, un groupe cyano, nitro, un reste alkyle, alkoxy, alkylthio, alkylsulfinyle ou alkylsulfonyle ayant chacun 1 à 6 atomes de carbone et dont chacun est éventuellement substitué par 1 à 5 atomes d'halogène.

3. Composés de formule (VIII) suivant la revendication 1, formule dans laquelle
A est un reste méthyle ou éthyle,
R¹, R², R³ et R⁴ sont identiques ou différents et représentent chacun, indépendamment l'un de l'autre, l'hydrogène, le fluor, le chlore, le brome, un groupe cyano, nitro, un reste méthyle, éthyle, n-propyle ou
isopropyle, n-butyle, isobutyle, butyle secondaire ou butyle tertiaire, méthoxy, éthoxy, n-propoxy ou iso-propoxy, méthylthio, éthylthio, méthylsulfinyle, éthylsulfinyle, méthylsulfonyle ou éthylsulfonyle, trifluorométhyle, trifluoréthylène, difluorométhoxy, trifluorométhoxy, difluorochlorométhoxy, trifluor-éthoxy, difluorométhylthio, difluorochlorométhylthio, trifluorométhylthio, trifluorométhylsulfinyle ou trifluorométhylsulfonyle.

4. Procédé de production de composés de formule (VIII) tels que définis dans la revendication 1, **caractérisé en ce que** :
h) on fait réagir des mono-oximes de benzofuranne-dione de formule 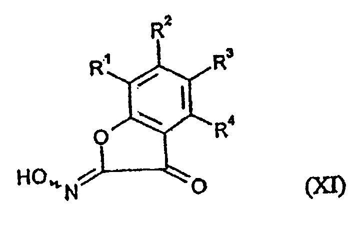 avec une alkoxyamine de formule (IV)
A-O-NH₂ (IV)
éventuellement en présence d'un diluant et, le cas échéant, en présence d'un accepteur d'acide, ou **en ce que** :
p) on fait réagir des dioximes de benzofuranne-dione de formule 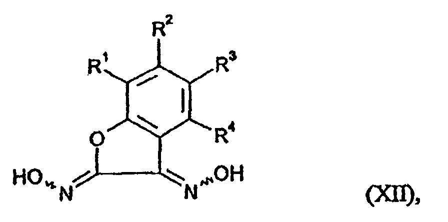 dans laquelle
R¹, R², R³ et R⁴ ont les définitions indiquées dans la revendication 1,
avec un agent d'alkylation de formule (VI)
A-X (VI)
éventuellement en présence d'un diluant, et, le cas échéant, en présence d'une base.
